# EUROPÄISCHE PATENTANMELDUNG

(11) **EP 3 786 149 A1**
(43) Veröffentlichungstag der Anmeldung: **03.03.2021**
(21) Anmeldenummer: 19193545.1
(22) Anmeldetag: 26.08.2019
(51) Int. Cl.: C07C 69/587, C11B 9/00

(54) **TERPENESTER, IHRE HERSTELLUNG UND VERWENDUNG ALS RIECH- UND GESCHMACKSTOFFE**

(71) Anmelder: Miltitz Aromatics GmbH, 06803 Bitterfeld-Wolfen (DE)
(72) Erfinder: GEBAUER, Helmut, 81479 München (US); PETRI, Andreas, 04155 Leipzig (DE); MÜLLER, Stefan, 04105 Leipzig (DE); HAUSHÄLTER, Julia, 04207 Leipzig (DE)
(74) Vertreter: Weidner Stern Jeschke

(57) **Zusammenfassung**

Die Erfindung betrifft eine Verbindung der Formel (I), 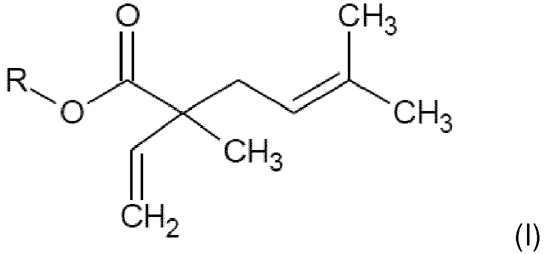 wobei R eine lineare oder verzweigte C₂- bis C₈-Alkylgruppe oder C₂- bis C₈-Alkenylgruppe oder eine C₃- bis C₈-Cycloalkylgruppe ist sowie Verfahren zur Herstellung einer solchen Verbindung und die Verwendung einer Verbindung der Formel (I) als Riech- oder Geschmackstoff, wobei R eine lineare oder verzweigte C₁- bis C₈-Alkylgruppe oder C₂- bis C₈-Alkenylgruppe oder eine C₃- bis C₈-Cycloalkylgruppe ist.

## Beschreibung

Die Erfindung betrifft Ester mit einer ungewöhnlichen Terpenstruktur, sowie deren Herstellung und Verwendung als Riech- oder Geschmackstoffe.

Terpenoide Ester wie Geranyl- und Linalylacetat werden in großen Mengen in der Riech- und Geschmackstoffindustrie eingesetzt. Da das Terpengerüst aus der Natur kommt, verleiht es auch den Parfüm- und Geschmackstoffmischungen den gewünschten natürlichen Charakter.

Trotz einer Vielzahl bereits vorhandener Stoffe besteht jedoch nach wie vor Bedarf an neuen Duftrichtungen, die der Kreativität der Parfümeure und Flavoristen weiteren Raum bieten.

Aufgabe der Erfindung war es daher, Stoffe aufzufinden, die dem Wunsch nach neuen natürlichen Duft- und Geschmacksbildern entsprechen.

Erfindungsgemäß wird die Aufgabe gelöst durch eine Verbindung der Formel (I), 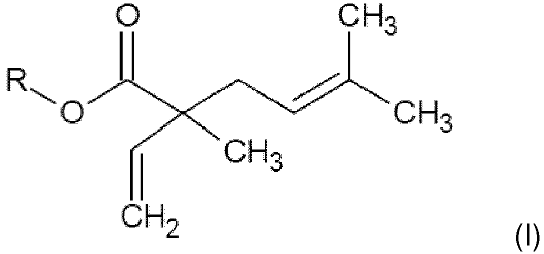 wobei R eine lineare oder verzweigte C₂- bis C₈-Alkylgruppe oder C₂- bis C₈-Alkenylgruppe oder eine C₃- bis C₈-Cycloalkylgruppe ist.

Bevorzugt ist R der Verbindung (I) eine Ethyl-, *n*-Propyl- oder *i*-Propyl-Gruppe.

In einer Ausführungsform der Erfindung wird die Aufgabe durch ein Verfahren zur Herstellung der Verbindung (I) mittels Veresterung gelöst.

Bevorzugt wird ein entsprechender Halogenkohlenwasserstoff oder ein entsprechender Alkohol verestert.

Besonders bevorzugt wird die Veresterung in Aceton durchgeführt.

Weiter bevorzugt besitzt das Verfahren die folgenden Schritte:
i. Umsetzen von 2-Methyl-2-butennitril oder 2-Methyl-3-butennitril mit Prenylchlorid zu 2,5-Dimethyl-2-vinyl-4-hexennitril;
ii. Verseifen des in Schritt (i) entstandenen 2,5-Dimethyl-2-vinyl-4-hexennitrils zu 2,5-Dimethyl-2-vinyl-4-hexensäure oder einem Salz der 2,5-Dimethyl-2-vinyl-4-hexensäure;
iii. Veresterung der in Schritt (ii) erhaltenen Verbindung mit dem entsprechenden Halogenalkan oder dem entsprechenden Alkohol.

Bevorzugt wird die Verseifung in Ethylenglykol durchgeführt.

Weiter bevorzugt wird KOH für die Verseifung verwendet.

Die Erfindung betrifft außerdem die Verwendung einer Verbindung der Formel (I) als Riech- oder Geschmackstoff, 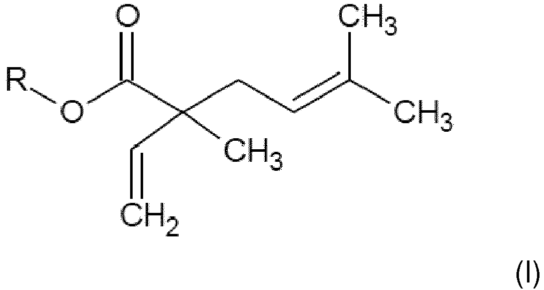 wobei R eine lineare oder verzweigte C₁- bis C₈-Alkylgruppe oder C₂- bis C₈-Alkenylgruppe oder eine C₃- bis C₈-Cycloalkylgruppe ist.

Bei Verwendung der Verbindung (I) ist R bevorzugt eine Methylgruppe.

Die Ester der Verbindung (I) weisen eine ungewöhnliche Terpenstruktur auf.

Der entsprechende Methylester (I), bei welchem R eine Methylgruppe ist, ist bereits bekannt, allerdings ohne Geruchsbeschreibung. Er wurde in einem nur labortauglichen Verfahren mit Hilfe von Lithiumorganylen und Diazomethan hergestellt, wohl für die pharmazeutische Forschung (J. Am. Chem. Soc. 1976, Vol. 98, 16, 4925 - 4935).

In einer Ausführungsform ist die Gruppe R der Verbindung (I) eine lineare oder verzweigte C₂- bis C₈-Alkylgruppe. In einer weiteren Ausführungsform ist die Gruppe R der Verbindung (I) eine lineare oder verzweigte C₂- bis C₈-Alkenylgruppe. In einer anderen Ausführungsform ist R der Verbindung (I) eine lineare oder verzweigte C₂- bis C₆-Alkylgruppe oder C₂- bis C₆-Alkenylgruppe oder eine C₃- bis C₆-Cycloalkylgruppe.

In einer weiteren Ausführungsform wird das Verfahren zur Herstellung der Verbindung (I) in einem organisch/alkalischen 2-Phasensystem in Gegenwart eines Phasentransferkatalysators durchgeführt.

Die erfindungsgemäßen Verbindungen sind durch Verseifung und Veresterung des zugrunde liegenden Nitrils zugänglich. Dieses wird gemäß EP 0135719 A1 durch Phasentransfer-katalysierte Alkylierung des großtechnisch verfügbaren 2-Methyl-2-butennitrils oder 2-Methyl-3-butennitrils mit Prenylchlorid hergestellt. Es werden pro Mol Nitril etwa 1-1,2 Mol Prenylchlorid eingesetzt.

Zur Phasentransfer-katalysierte Alkylierung verwendet man ein organisch/alkalisches 2-Phasensystem, gebildet aus einem organischen, mit Wasser nicht mischbaren inerten Lösungsmittel und einer 5-50 Gew.-%igen wässrigen Lösung oder in fester Form vorliegendem Alkalimetallhydroxid. Als Phasentransferkatalysatoren werden beispielsweise Kronenether, quartäre Ammonium- und Phosphoniumsalze eingesetzt in Mengen von 0,5-5 Mol%, bezogen auf Prenylchlorid. Die Reaktionstemperaturen betragen 20-150 °C, bevorzugt 60-80°C.

Die Verseifung von 2,5-Dimethyl-2-vinyl-4-hexennitril zu 2,5-Dimethyl-2-vinyl-4-hexensäure gelingt durch Umsetzung mit KOH in Ethylenglykol und nachfolgendes Ansäuern mit Schwefelsäure. Dabei wird das Nitril mit der zweifachen molaren Menge KOH, gelöst in Ethylenglykol, zum Sieden erhitzt, bis die Ammoniakentwicklung beendet ist. Nach Wasserzugabe und Neutralisierung wird die freigesetzte Säure mit einem organischen Lösungsmittel extrahiert.

Die anschließende Veresterung erfolgt durch Umsetzung der Säure mit der 1- bis 2-fachen Menge des entsprechenden Halogenalkans in Gegenwart einer Base oder durch Umsetzung der Säure mit der 1- bis 4-fachen Menge des entsprechenden Alkohols in Gegenwart einer Säure. Die Synthese gelingt auch durch Umesterung eines höheren Alkohols.

Aus natürlichen Ressourcen ist diese Terpenstruktur ökonomisch nicht gewinnbar: Der entsprechende Aldehyd wurde in geringen Mengen in den teuren Neroli- und Verbenaölen gefunden, der Alkohol in Spuren in den Blättern der Ficus- und Teepflanze.

Der vorstehend beschriebene Syntheseweg stellt das bevorzugte Verfahren zur Herstellung der erfindungsgemäßen Ester dar, das dadurch gekennzeichnet ist, dass
a) 2-Methyl-2-butennitril oder 2-Methyl-3-butennitril und Prenylchlorid im Eintopfverfahren in einem organisch/alkalischen 2-Phasensystem in Gegenwart eines Phasentransferkatalysators umgesetzt werden,
b) das entstandene Nitril alkalisch verseift wird und
c) die entstandene Säure mit dem entsprechenden Halogenalkan oder Alkohol verestert wird.

Ethyl, 2,5-Dimethyl-2-vinyl-4-hexenoat ist ein Riechstoff mit starker natürlicher Ausstrahlung, der sowohl fruchtige Anklänge etwa von Zitrone beinhaltet, aber in erster Linie blumige Aspekte aufweist. Man riecht Rose, Geranium, Bergamotte. Bevorzugtes Einsatzgebiet sind daher fruchtig-blumige Kompositionen.

n-Propyl, 2,5-Dimethyl-2-vinyl-4-hexenoat weist eine starke balsamische Duftnote auf, die an schwere Blütendüfte erinnert. Die fruchtige Komponete tritt hier etwas in den Hintergrund.

i-Propyl, 2,5-Dimethyl-2-vinyl-4-hexenoat, ein ebenfalls sehr starker Duft, sieht die agrumige Note im Vordergrund. Natürlich ist auch der blumige Aspekt wieder vorhanden, an ein Lavendelfeld erinnernd.

Methyl, 2,5-Dimethyl-2-vinyl-4-hexenoat hat einen sehr starken natürlichen Geruch mit voller herber würziger und fruchtiger Note, erinnernd an Bergamotte, Grapefruit und vor allem Koriander. Der Ester riecht bereits wie eine Duftstoffmischung.

Die erfindungsgemäßen Riechstoffe können in der Feinparfümerie und zur Parfümierung kosmetischer und technischer Produkte allein und insbesondere in Kombination mit anderen Riechstoffen eingesetzt werden. Sie entsprechen dem Wunsch der Parfümerie nach neuen natürlichen Duftnoten. Ebenso können die Ester in Geschmackstoff-Kompositionen verwendet werden.

Die Erfindung wird nun anhand von Beispielen näher erläutert:
Soweit sich im Folgenden Zahlenangaben auf die Zusammensetzung von Duftstoffmischungen beziehen, sollen darunter Gewichtsteile verstanden werden.

### Beispiel 1

### Herstellung von Ethyl, 2,5-Dimethyl-2-vinyl-4-hexenoat

In einem Sulfierkolben werden 288,0 g 2,5-Dimethyl-2-vinyl-4-hexensäure (1,712 mol) in 2100 ml Aceton gelöst. Zu der erhaltenen klaren, gelblichen Lösung werden portionsweise 354,9 g K₂CO₃ (2,568 mol) gegeben. Die Innentemperatur steigt dabei von 21 °C auf 31 °C an; gegen Ende der Zugabe wird eine weisse Suspension mit ungelöstem Feststoff erhalten. Nach Abkühlen des Kolbeninhalts auf 20°C werden über einen Zeitraum von 20 min 400,5 g lodethan (2,568 mol) zugegeben und die erhaltene Reaktionsmischung wird bei 21 - 23 °C über einen Zeitraum von 21 h gerührt. Im Anschluss daran wird die Reaktionsmischung mit 1,8 Liter Wasser versetzt und bei Raumtemperatur gerührt. Die Phasen werden getrennt und die wässrige Phase wird mit Toluol rückextrahiert. Nach erneuter Phasentrennung werden die vereinigten organischen Phasen im Vakuum vom Lösungsmittel befreit. Der dadurch erhaltene Rückstand wird erneut mit Wasser gefolgt von gesättigter Kochsalz-Lösung gewaschen. Nach Trocknung über Natriumsulfat wird das Ethyl, 2,5-Dimethyl-2-vinyl-4-hexenoat als Rohprodukt erhalten.

Das Rohprodukt Ethyl, 2,5-Dimethyl-2-vinyl-4-hexenoat wird bei einem Vakuum von 0,5 - 1 mbar über eine Metall-Füllkörperkolonne fraktioniert. Unter diesen Bedingungen destilliert das Zielprodukt bei einer Kopftemperatur von 58 - 64°C über. Es werden 212 g gereinigtes Ethyl, 2,5-Dimethyl-2-vinyl-4-hexenoat erhalten.

### Beispiel 2

### Herstellung von n-Propyl, 2,5-Dimethyl-2-vinyl-4-hexenoat

In einem Sulfierkolben werden 329,4 g 2,5-Dimethyl-2-vinyl-4-hexensäure (1,958 mol) in 2400 ml Aceton gelöst. Zu der erhaltenen klaren, gelblichen Lösung werden portionsweise 406,0 g K₂CO₃ (2,937 mol) gegeben. Die Innentemperatur steigt dabei von 21 °C auf 30°C an; gegen Ende der Zugabe wird eine weisse Suspension mit ungelöstem Feststoff erhalten. Nach Abkühlen des Kolbeninhalts auf 20°C werden über einen Zeitraum von 20 min 499,3 g 1-lodpropan (2,937 mol) zugegeben und die erhaltene Reaktionsmischung wird bei 21 - 23 °C über einen Zeitraum von 21 h gerührt. Im Anschluss daran wird die Reaktionsmischung mit 2,1 Liter Wasser versetzt und bei Raumtemperatur gerührt. Die Phasen werden getrennt und die wässrige Phase wird mit Toluol rückextrahiert. Nach erneuter Phasentrennung werden die vereinigten organischen Phasen im Vakuum vom Lösungsmittel befreit. Der dadurch erhaltene Rückstand wird erneut mit Wasser gefolgt von gesättigter Kochsalz-Lösung gewaschen. Nach Trocknung über Natriumsulfat wird das *n*-Propyl, 2,5-Dimethyl-2-vinyl-4-hexenoat als Rohprodukt erhalten.

Das Rohprodukt *n*-Propyl, 2,5-Dimethyl-2-vinyl-4-hexenoat wird bei einem Vakuum von 0,5 - 1 mbar über eine Metall-Füllkörperkolonne fraktioniert. Unter diesen Bedingungen destilliert das Zielprodukt bei einer Kopftemperatur von 62 - 67 °C über. Es werden 242,5 g gereinigtes *n*-Propyl, 2,5-Dimethyl-2-vinyl-4-hexenoat erhalten.

### Beispiel 3

### Herstellung von i-Propyl, 2,5-Dimethyl-2-vinyl-4-hexenoat

In einem Sulfierkolben werden 457,4 g 2,5-Dimethyl-2-vinyl-4-hexensäure (2,719 mol) in 3300 ml Aceton gelöst. Zu der erhaltenen klaren, gelblichen Lösung werden portionsweise 563,7 g K₂CO₃ (4,079 mol) gegeben. Die Innentemperatur steigt dabei von 21 °C auf 31 °C an; gegen Ende der Zugabe wird eine weisse Suspension mit ungelöstem Feststoff erhalten. Nach Abkühlen des Kolbeninhalts auf 20°C werden über einen Zeitraum von 20 min 724,3 g 2-lodpropan (4,261 mol) zugegeben und die erhaltene Reaktionsmischung wird bei 33 - 35°C über einen Zeitraum von 48 h gerührt. Im Anschluss daran wird die Reaktionsmischung mit 3 Litern Wasser versetzt und bei Raumtemperatur gerührt. Die Phasen werden getrennt und die wässrige Phase wird mit Toluol rückextrahiert. Nach erneuter Phasentrennung werden die vereinigten organischen Phasen im Vakuum vom Lösungsmittel befreit. Der dadurch erhaltene Rückstand wird erneut mit Wasser gefolgt von gesättigter Kochsalz-Lösung gewaschen. Nach Trocknung über Natriumsulfat wird das *i*-Propyl, 2,5-Dimethyl-2-vinyl-4-hexenoat als Rohprodukt erhalten.

Das Rohprodukt *i*-Propyl, 2,5-Dimethyl-2-vinyl-4-hexenoat wird bei einem Vakuum von 0,5 - 1 mbar über eine Metall-Füllkörperkolonne fraktioniert. Unter diesen Bedingungen destilliert das Zielprodukt bei einer Kopftemperatur von 61 - 68°C über. Es werden 235,6 g gereinigtes *i-*Propyl, 2,5-Dimethyl-2-vinyl-4-hexenoat erhalten.

### Beispiel 4

### Herstellung von i-Propyl, 2,5-Dimethyl-2-vinyl-4-hexenoat

In einem Rundkolben werden 50,5 g 2,5-Dimethyl-2-vinyl-4-hexensäure (0,300 mol) in 70 ml Isopropanol gelöst. Zur Lösung werden 12,2 g wasserfreies Calciumchlorid (0,110 mol) gefolgt von 2,86 g para-Toluolsulfonsäure-Monohydrat (0,015 mol) gegeben und die Reaktionsmischung wird bei 60°C über einen Zeitraum von 21 h gerührt. Mittels gaschromatographischer Analyse einer Reaktionsprobe konnte die Bildung des entsprechenden Esters *i*-Propyl, 2,5-Dimethyl-2-vinyl-4-hexenoat nachgewiesen werden.

### Beispiel 5

### Herstellung von Methyl, 2,5-Dimethyl-2-vinyl-4-hexenoat

In einem Sulfierkolben werden 476,7 g 2,5-Dimethyl-2-vinyl-4-hexensäure (2,834 mol) in 3400 ml Aceton gelöst. Zu der erhaltenen klaren, gelblichen Lösung werden portionsweise 587,5 g K₂CO₃ (4,251 mol) gegeben. Die Innentemperatur steigt dabei von 20°C auf 31 °C an; gegen Ende der Zugabe wird eine weisse Suspension mit ungelöstem Feststoff erhalten. Nach Abkühlen des Kolbeninhalts auf 20°C werden über einen Zeitraum von 20 min 704,2 g lodmethan (4,961 mol) zugegeben und die erhaltene Reaktionsmischung wird bei 21 - 23 °C über einen Zeitraum von 21 h gerührt. Im Anschluss daran wird die Reaktionsmischung mit 3 Liter Wasser versetzt und bei Raumtemperatur gerührt. Die Phasen werden getrennt und die wässrige Phase wird mit Toluol rückextrahiert. Nach erneuter Phasentrennung werden die vereinigten organischen Phasen im Vakuum vom Lösungsmittel befreit. Der dadurch erhaltene Rückstand wird erneut mit Wasser gefolgt von gesättigter Kochsalz-Lösung gewaschen. Nach Trocknung über Natriumsulfat wird das Methyl, 2,5-Dimethyl-2-vinyl-4-hexenoat als Rohprodukt erhalten.

Das Rohprodukt Methyl, 2,5-Dimethyl-2-vinyl-4-hexenoat wird bei einem Vakuum von 0,5 - 1 mbar über eine Metall-Füllkörperkolonne fraktioniert. Unter diesen Bedingungen destilliert das Zielprodukt bei einer Kopftemperatur von 52 - 58°C über. Es werden 351 g gereinigtes Methyl, 2,5-Dimethyl-2-vinyl-4-hexenoat erhalten.

### Beispiel 6

### Herstellung von 2,5-Dimethyl-2-vinyl-4-hexensäure

In einem Rundkolben werden 713,7 g Kaliumhydroxid (12,72 mol) vorgelegt und es werden 1900 g Monoethylenglykol zugegeben. Die Mischung wird erwärmt und bei einer Innentemperatur von 100 - 104°C werden über einen Zeitraum von 3 Stunden 474,4 g 2,5-Dimethyl-2-vinyl-4-hexennitril zugetropft. Die erhaltene Reaktionsmischung wird für 72 Stunden bei einer Innentemperatur von 104 bis 112 °C gerührt. Danach wird der Kolbeninhalt an eine Vakuum-Destillationsapparatur angeschlossen und die im Ansatz enthaltenen Leichtsieder sowie Teile des Monoethylenglykols im Vakuum entfernt. Der verbleibende Rückstand wird in 2 Liter Wasser aufgenommen und die erhaltene Lösung mit 25 Gew.-% iger H₂SO₄ auf einen pH-Wert von 3 eingestellt, wobei die Innentemperatur mittels Eiskühlung unter 18°C gehalten wurde.

Die erhaltene Mischung wird mit 1 Liter Wasser und 1 Liter Toluol versetzt und filtriert. Der Filterkuchen wird verworfen und mit dem erhaltenen zweiphasigen Filtrat wird eine Phasentrennung durchgeführt. Die obere organische Phase wird mit Wasser gewaschen und das Lösungsmittel im Vakuum entfernt. Es werden 487,1 g 2,5-Dimethyl-2-vinyl-4-hexensäure als klare, hellrote Flüssigkeit erhalten.

### Beispiel 7

### Verwendung von Ethyl, 2,5-Dimethyl-2-vinyl-4-hexenoat als Riechstoff. Süß-blumiges Parfümöl

| | a | b |
|---|---|---|
| Phenylethanol | 70 | 70 |
| Hydroxycitronellal | 200 | 200 |
| Verdylacetat Giv | 60 | 60 |
| Sandelholzöl | 55 | 55 |
| Jasmin synth. | 80 | 80 |
| Cyclohexadecenon | 40 | 40 |
| Citronellol | 60 | 60 |
| Ylang-ylang-Öl | 35 | 35 |
| γ-Methylionon | 50 | 50 |
| Benzylpropionat | 60 | 60 |
| Geraniol | 40 | 40 |
| Dipropylenglykol | 250 | -- |
| Ethyl, 2,5-Dimethyl-2-vinyl-4-hexenoat | -- | 250 |
| | 1000 | 1000 |

Die blumige Komposition gemäß a gewinnt durch Zugabe von 250 Gew.TI. Ethyl, 2,5-Dimethyl-2-vinyl-4-hexenoat an Strahlkraft und Natürlichkeit. Weiter werden die fruchtigen Nuancen verstärkt.

### Beispiel 8

Verwendung von *n*-Propyl, 2,5-Dimethyl-2-vinyl-4-hexenoat als Riechstoff.

### Parfümöl mit Fougère-Note

| | a | b | |
|---|---|---|---|
| Bergamottöl | 200 | 200 | |
| Cumarin | 220 | 220 | |
| Lavandinöl | 100 | 100 | |
| Vetiveröl | 20 | 20 | |
| Linalylacetat | 30 | 30 | |
| Terpinylacetat | 30 | 30 | |
| Phenylethanol | 50 | 50 | |
| Geraniumöl | 30 | 30 | |
| Patchouliöl | 30 | 30 | |
| Hydroxyambran MA | 20 | 20 | |
| Benzylacetat | 40 | 40 | |
| α-Amylzimtaldehyd | 30 | 30 | |
| Dipropylenglykol | 200 | -- | |
| n-Propyl, 2,5-Dimethyl-2-vinyl-4-hexenoat | | -- | 200 |
| | 1000 | 1000 | |

Der Duftkomplex gemäß a ist ein Fougere-Typ mit vorherrschender Cumarinnote. Durch Zusatz von 200 Gew.TI. n-Propyl, 2,5-Dimethyl-2-vinyl-4-hexenoat (gemäß b) tritt der warm-würzige Charakter in den Hintergrund.

Er erhält eine balsamische Komponente, die den natürlichen Geruchseffekt der Base erhöht.

### Beispiel 9

Verwendung von *i*-Propyl, 2,5-Dimethyl-2-vinyl-4-hexenoat als Riechstoff.

### Parfümöl mit Citrusnote

| | a | b |
|---|---|---|
| Agrumenaldehyd hell Sym. | 20 | 20 |
| Decanal | 10 | 10 |
| Dihydromyrcenol | 425 | 425 |
| Limette dest. | 40 | 40 |
| Orangenölterpene | 360 | 360 |
| Cyclohexadecenon | 50 | 50 |
| Hydroxyambran MA | 25 | 25 |
| tert.-Butylhydroxyanisol | 10 | 10 |
| Dipropylenglykol | 60 | -- |
| i-Propyl, 2,5-Dimethyl-2-vinyl-4-hexenoat | -- | 60 |
| | 1000 | 1000 |

i-Propyl, 2,5-Dimethyl-2-vinyl-4-hexenoat verstärkt in diesem Parfümöl die frische, agrumige Kopfnote, wobei seine blumige Nebennote der Komposition mehr Natürlichkeit und Komplexität verleiht.

### Beispiel 10

Verwendung von Methyl, 2,5-Dimethyl-2-vinyl-4-hexenoat als Riechstoff. Würzig-holziges, blumiges Parfümöl

| | a | b | |
|---|---|---|---|
| Hydroxyambran 10 Gew.-% MA | | 200 | 200 |
| Galaxolide 50 IFF | 100 | 100 | |
| Hedione Firm. | 100 | 100 | |
| Majantol Sym | 100 | 100 | |
| Dihydromyrcenol | 100 | 100 | |
| Geranyltiglat MA | 200 | 200 | |
| Cetalox 10 Gew.-% Firm. | | 60 | 60 |
| Galbex Firm. | 40 | 40 | |
| Triplal IFF | 20 | 20 | |
| β-Damascon 10 Gew.-% | | 20 | 20 |
| Dipropylenglykol | -- | 60 | |
| Methyl, 2,5-Dimethyl-2-vinyl-4-hexenoat | 60 | -- | |
| | 1000 | 1000 | |

Das Parfümöl erfährt durch Zugabe von 60 Gew.TI. Methyl, 2,5-Dimethyl-2-vinyl-4-hexenoat anstelle von Dipropylenglykol enorme Strahlkraft, Natürlichkeit und Abrundung.

## Patentansprüche

1. Verbindung der Formel (I), 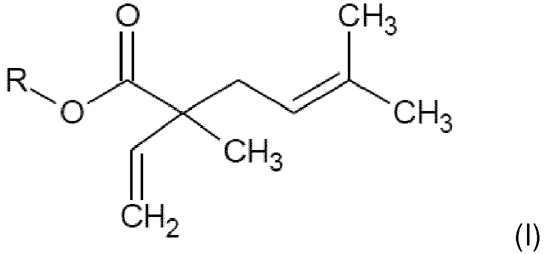 wobei R eine lineare oder verzweigte C₂- bis C₈-Alkylgruppe oder C₂- bis C₈-Alkenylgruppe oder eine C₃- bis C₈-Cycloalkylgruppe ist.

2. Verbindung nach Anspruch 1, **dadurch gekennzeichnet, dass** R eine Ethyl-, *n*-Propyl- oder *i*-Propyl-Gruppe ist.

3. Verfahren zur Herstellung der Verbindung (I) nach Anspruch 1 oder 2, **dadurch gekennzeichnet, dass** die Verbindung (I) mittels Veresterung hergestellt wird.

4. Verfahren nach Anspruch 3, **dadurch gekennzeichnet, dass** ein entsprechender Halogenkohlenwasserstoff oder ein entsprechender Alkohol verestert wird.

5. Verfahren nach Anspruch 3 oder 4, **dadurch gekennzeichnet, dass** die Veresterung in Aceton durchgeführt wird.

6. Verfahren nach einem der Ansprüche 3 bis 5, **gekennzeichnet durch** die folgenden Schritte:
i. Umsetzen von 2-Methyl-2-butennitril oder 2-Methyl-3-butennitril mit Prenylchlorid zu 2,5-Dimethyl-2-vinyl-4-hexennitril;
ii. Verseifen des in Schritt (i) entstandenen 2,5-Dimethyl-2-vinyl-4-hexennitrils zu 2,5-Dimethyl-2-vinyl-4-hexensäure oder einem Salz der 2,5-Dimethyl-2-vinyl-4-hexensäure;
iii. Veresterung der in Schritt (ii) erhaltenen Verbindung mit dem entsprechenden Halogenalkan oder dem entsprechenden Alkohol.

7. Verfahren nach Anspruch 6, **dadurch gekennzeichnet, dass** die Verseifung in Ethylenglykol durchgeführt wird.

8. Verfahren nach Anspruch 6 oder Anspruch 7, **dadurch gekennzeichnet, dass** KOH für die Verseifung verwendet wird.

9. Verwendung einer Verbindung der Formel (I) als Riech- oder Geschmackstoff, 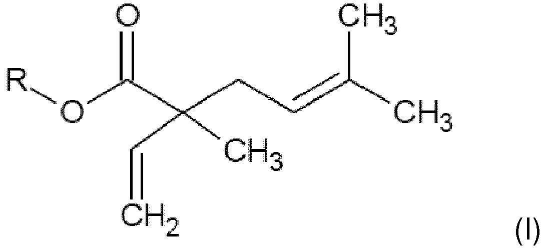 wobei R eine lineare oder verzweigte C₁- bis C₈-Alkylgruppe oder C₂- bis C₈-Alkenylgruppe oder eine C₃- bis C₈-Cycloalkylgruppe ist.

10. Verwendung der Verbindung (I) nach Anspruch 9, **dadurch gekennzeichnet, dass** R eine Methylgruppe ist.

## Geänderte Patentansprüche

### Geänderte Patentansprüche gemäss Regel 137(2) EPÜ.

1. Verbindung der Formel (I), 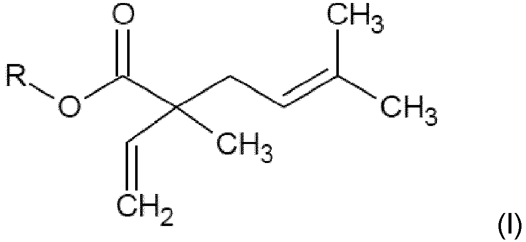 wobei R eine *n*-Propyl- oder *i*-Propyl-Gruppe ist.

2. Verfahren zur Herstellung der Verbindung der Formel (I) 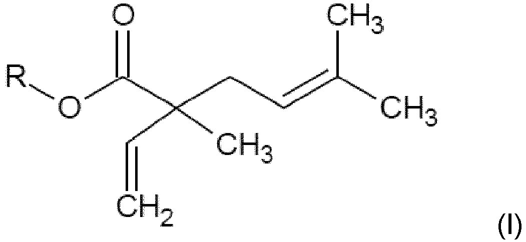 wobei R eine lineare oder verzweigte C₂- bis C₈-Alkylgruppe oder C₂- bis C₈-Alkenylgruppe oder eine C₃- bis C₈-Cycloalkylgruppe ist, **dadurch gekennzeichnet, dass** die Verbindung (I) mittels Veresterung hergestellt wird.

3. Verfahren nach Anspruch 2, **dadurch gekennzeichnet, dass** ein entsprechender Halogenkohlenwasserstoff oder ein entsprechender Alkohol verestert wird.

4. Verfahren nach Anspruch 2 oder 3, **dadurch gekennzeichnet, dass** die Veresterung in Aceton durchgeführt wird.

5. Verfahren nach einem der Ansprüche 2 bis 4, **gekennzeichnet durch** die folgenden Schritte:
i. Umsetzen von 2-Methyl-2-butennitril oder 2-Methyl-3-butennitril mit Prenylchlorid zu 2,5-Dimethyl-2-vinyl-4-hexennitril;
ii. Verseifen des in Schritt (i) entstandenen 2,5-Dimethyl-2-vinyl-4-hexennitrils zu 2,5-Dimethyl-2-vinyl-4-hexensäure oder einem Salz der 2,5-Dimethyl-2-vinyl-4-hexensäure;
iii. Veresterung der in Schritt (ii) erhaltenen Verbindung mit dem entsprechenden Halogenalkan oder dem entsprechenden Alkohol.

6. Verfahren nach Anspruch 5, **dadurch gekennzeichnet, dass** die Verseifung in Ethylenglykol durchgeführt wird.

7. Verfahren nach Anspruch 5 oder Anspruch 6, **dadurch gekennzeichnet, dass** KOH für die Verseifung verwendet wird.

8. Verwendung einer Verbindung der Formel (I) als Riech- oder Geschmackstoff, 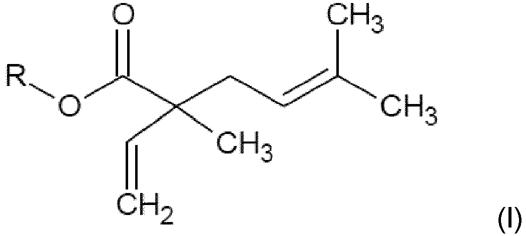 wobei R eine Methyl-, Ethyl-, *n*-Propyl- oder *i*-Propylgruppe ist.
